# EUROPÄISCHE PATENTSCHRIFT

(11) **EP 0 266 611 B1**
(45) Veröffentlichungstag und Bekanntmachung des Hinweises auf die Patenterteilung: **23.06.1993**
(21) Anmeldenummer: 87115241.9
(22) Anmeldetag: 19.10.1987
(51) Int. Cl.: G01N 33/36

(54) **Vorrichtung zur automatischen Bestimmung von Kenngrössen von textilem Prüfgut**
Device for the automatic determination of data concerning a textile product
Appareil pour la détermination automatique de caractéristiques d'un produit textile

(30) Priorität: 06.11.1986 CH 4433/86
(43) Veröffentlichungstag der Anmeldung: 11.05.1988
(73) Patentinhaber: ZELLWEGER USTER AG, CH-8610 Uster (CH)
(72) Erfinder: Heusser, Eduard, CH-8610 Uster (CH)

(56) Entgegenhaltungen:
- EP-A- 0 129 076
- US-A- 3 788 138

## Beschreibung

Die Erfindung betrifft eine Vorrichtung zur automatischen Bestimmung von Kenngrössen von textilem Prüfgut, wie Garnen, Vorgarnen und Bändern, mit einer eine Führungseinrichtung, ein Messorgan, eine Vorschubeinrichtung und ein Absaugorgan für das Prüfgut enthaltenden Messeinheit, deren Messorgan durch einen Messkamm mit Messspalten verschiedener Breite gebildet ist.

In den Labors von Textilbetrieben, vornehmlich von Spinnereien, werden im Rahmen der betrieblichen Qualitätskontrolle Stichprobenprüfungen zur Bestimmung gewisser textiler Parameter, wie beispielsweise der Masseschwankungen und anderer davon abgeleiteter Kenngrössen vorgenommen. Dazu dienen sogenannte Gleichmässigkeitsprüfer, wie sie beispielsweise von der Anmelderin der vorliegenden Patentanmeldung unter der Bezeichnung USTER TESTER (USTER eingetragenes Warenzeichen der Zellweger Uster AG) weltweit vertrieben werden. Bei diesem bekannten Gleichmässigkeitsprüfer stellt jeder Messspalt den Luftspalt eines Kondensators dar, und die Bestimmung der genannten Kenngrössen erfolgt kapazitiv.

Zur Bestimmung der Masseschwankungen des Prüfguts wird dieses durch die Vorschubeinrichtung durch einen der Messspalte des Messkamms gezogen, wobei die Wahl des jeweiligen Messspalts von der Feinheit oder Garnnummer des Prüfguts bestimmt ist. Der Messeinheit ist ein Einlegemechanismus für das Prüfgut zugeordnet, welcher das letztere ergreift und in den vorbestimmten Messspalt einlegt.

Da zumindest am Beginn jeder Messung bei der Messeinheit ein Nullabgleich vorgenommen oder der Nullabgleich überprüft werden muss, muss das bereits in den Messspalt eingelegte Prüfgut kurzfristig wieder aus diesem entfernt werden. Dies erfolgte bisher von Hand durch die jeweilige Bedienungsperson.

Beim Bestreben der weiteren Verbesserung der bekannten Gleichmässigkeitsprüfer im Hinblick auf die Erhöhung des Bedienungskomforts und der Zuverlässigkeit hat sich gezeigt, dass die von Hand vorzunehmende Entfernung des Prüfguts aus dem Messspalt beim Nullabgleich einerseits eine gewisse Fehlerquelle darstellt und anderseits nicht bedienungsfreundlich ist und daher bei einem verbesserten Gleichmässigkeitsprüfer vermieden werden sollte.

Diese Aufgabe wird durch die im kennzeichnenden Teil von Anspruch 1 angegebenen Merkmale gelöst.

Durch den auf dem Verstellorgan angeordneten Garnheber wird das Prüfgut beim Nullabgleich maschinell aus dem Messspalt entfernt und anschliessend wieder in diesen eingelegt. Dadurch wird nicht nur die Bedienungsperson entlastet, sondern es werden auch mögliche Bedienungsfehler vermieden, da das Prüfgut automatisch wieder in den richtigen Messspalt gelangt.

Im folgenden wird die Erfingung anhand eines Ausführungsbeispiels und der Zeichnungen näher dargestellt; es zeigen:
- Fig. 1: eine Perspektivdarstellung eines Gleichmässigkeitsprüfers zur Bestimmung der Masseschwankungen von Stapelfasergarnen,
- Fig. 2: eine Perspektivdarstellung der wesentlichen Teile des Messorgans der Messeinheit des Gleichmässigkeitsprüfers,
- Fig. 3: eine Frontansicht eines Teils der Messeinheit, und
- Fig. 4: eine Ansicht in Richtung des Pfeils IV von Fig. 3, bei weggelassener oberer Gehäuseplatte.

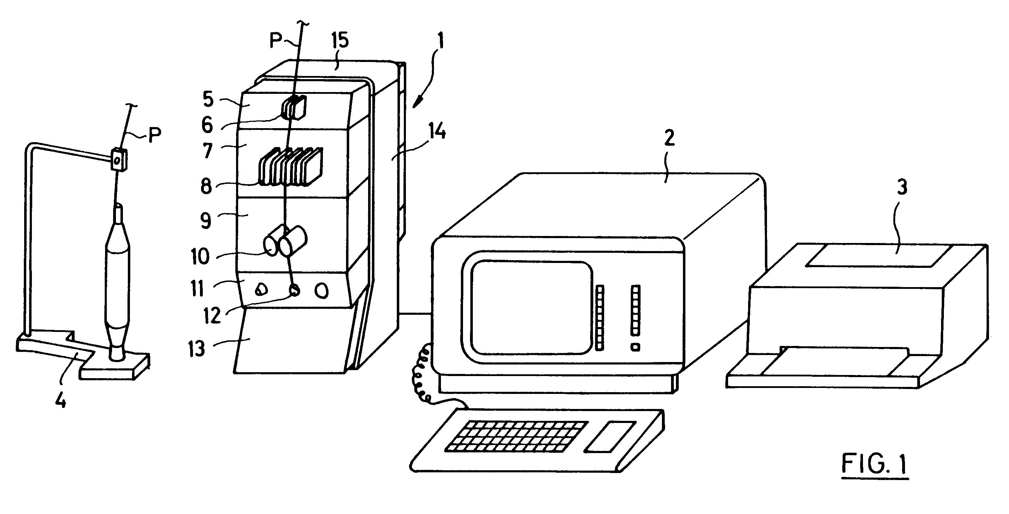

Der in Fig. 1 dargestellte Gleichmässigkeitsprüfer zur Bestimmung der Masseschwankungen von textilem Prüfgut, wie Garnen, Vorgarnen oder Bändern aus Stapelfasern, besteht darstellungsgemäss aus einer Messeinheit 1, aus einer Auswerteeinheit 2, aus einer Ausgabeeinheit 3 und aus einem Gestell 4 für die Aufmachungseinheiten mit dem Prüfgut P, das sind beispielsweise Garn- oder Vorgarnspulen. Gleichmässigkeitsprüfer dieser Art sind bekannt und werden beispielsweise von der Anmelderin der vorliegenden Patentanmeldung unter der Bezeichnung USTER TESTER (USTER eingetragenes Warenzeichen der Zellweger Uster AG) weltweit vertrieben.

Die Messeinheit 1 für das Prüfgut P besteht darstellungsgemäss aus mehreren Modulen, welche in Laufrichtung des Prüfguts P, das ist in der Figur von oben nach unten, wie folgt angeordnet sind: Zuerst ein Modul 5 mit einer Fadenführungseinrichtung 6, beispielsweise einer Fadenbremse, anschliessend ein Modul 7 mit einem Messorgan 8, anschliessend ein Modul 9 mit einer Vorschubeinrichtung 10 und anschliessend ein Modul 11 mit einer Absaugdüse 12. Das unterste Modul 11 ist auf einen Sockel 13 aufgesetzt und alle genannten Module 5, 7, 9 und 11 sowie der Sockel 13 sind in einem Rahmen 14 mit einem bügelartigen Oberteil 15 angeordnet und von diesem Rahmen gehalten.

Das Messorgan 8, durch welches das Prüfgut P durch die durch ein Walzenpaar gebildete Vorschubeinrichtung 10 gezogen wird, ist ein sogenanntes kapazitives Messorgan. Dieses ist in den US Patenten 3 754 172, 3 788 138 und 3 805 607 beschrieben, auf deren Offenbarung hiermit Bezug genommen wird. Die Absaugdüse 12 ist von dem bereits genannten USTER TESTER bekannt und wird hier nicht näher beschrieben.

Die Auswerteeinheit 2 enthält unter anderem einen Analog-/Digitalwandler und einen Rechner und ist darstellungsgemäss mit einem Bildschirm kombiniert. Die vom Messorgan 8 laufend erzeugten elektrischen Signale werden vom Rechner der Auswerteeinheit 2 verarbeitet und in geeigneter Form in einem in der Auswerteeinheit 2 integrierten Speicher gespeichert und können vor ihrem Ausdruck auf dem Ausgabeeinheit 3 bildenden Drucker auf dem Bildschirm dargestellt werden. Dies hat der Vorteil, dass man alle anfallenden Daten vorerst auf dem Bildschirm zur Anzeige bringen und nur ausgewählte Daten für den Ausdruck auf dem Drucker 3 bestimmen kann.

Es sei noch darauf hingewiesen, dass die Signalverarbeitung in der Auswerteeinheit 2 aus drei Hauptkomponenten besteht, nämlich aus dem Spektrographen, für das sogenannte Spektrogramm (Wellenlängenspektrum der Masseschwankungen), aus dem Imperfection Indicator, welcher Grenzwertüberschreitungen der Masse zählt, und aus dem eigentlichen Auswerteteil für die Bestimmung des sogenannten Variationskoeffizienten und der Längenvariationskurve. Alle diese Kenngrössen sind von dem schon genannten USTER TESTER her bekannt.

Wenn mit dem dargestellten Gleichmässigkeitsprüfer die Masseschwankungen von Filamentgarnen bestimmt werden sollen, dann muss eine Messeinheit 1 mit einem anderen Aufbau verwendet werden, welche gegenüber der in der Figur dargestellten Messeinheit 1 folgende Abweichungen aufweist:
- Es wird ein anderes Messorgan 8 benötigt, welches aber ebenfalls ein kapazitives Messorgan ist.
- Die Vorschubeinrichtung 10 muss in Laufrichtung des Prüfguts P vor dem Messorgan 8 angeordnet sein.
- Es wird eine spezielle Absaugdüse 12 benötigt, welche dem Filamentgarn den für die Prüfung erforderlichen Drall verleiht. Bezüglich dieser Absaugdüse wird hiermit auf das US-Patent Nr. 3 951 321 verwiesen.

Durch Einfügen weiterer Module in die Messeinheit 1 kann diese für die Bestimmung weiterer Parameter des Prüfguts P ausgebaut werden. So kann beispielsweise mit einem zusätzlichen Modul mit einem Messorgan für die Haarigkeit des Prüfguts P zusätzlich zu den Masseschwankungen und in einem einzigen Durchlauf des Prüfguts P durch die Messeinheit 1, dessen Haarigkeit bestimmt werden. Bezüglich dieser Ausbaumöglichkeit der Messeinheit 1 wird auf das deutsche Gebrauchs muster 8708187 der Anmelderin der vorliegenden Patentanmeldung verwiesen.

Das Prüfgut P ist beim Durchlauf durch die Messeinheit 1 im Verlauf seiner Prüfung zwischen der Fadenführungseinrichtung 6 und der Absaugdüse 12 gespannt, wobei die beiden die Vorschubeinrichtung 10 bildenden Walzen so angeordnet sind, dass durch diese keine Auslenkung des Prüfguts P aus seiner geraden Bahn erfolgt.

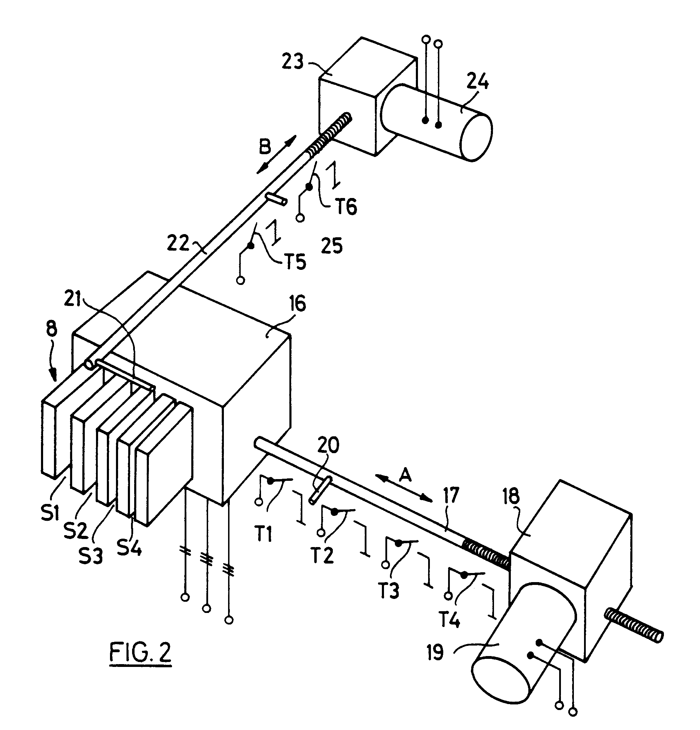

Fig. 2 zeigt eine perspektivische Darstellung der wesentlichsten Teile des Messorgans 8 der Messeinheit 1 des Gleichmässigkeitsprüfers von Fig. 1. Wie schon erwähnt wurde, ist das Messorgan ein sogenanntes kapazitives Messorgan und besteht aus einem Messkamm mit plattenartigen Vorsprüngen, welche Messspalte S1, S2, S3, S4 mit verschiedener Spaltbreite definieren. Je nach der Garnnummer des Prüfguts durchläuft dieses bei der Prüfung einen bestimmten Messspalt S1, S2, S3 oder S4.

Um zu gewährleisten, dass beim Einlegen des Prüfguts in die verschiedenen Messpalte S1 bis S4 keine verschiedenen Auslenkungen des Prüfguts aus seiner normalen Bahn erfolgen, ist der Messkamm 8 quer zur Laufrichtung des Prüfguts verschiebbar ausgebildet, wobei die Achse jedes Messspalts S1 bis S4 auf eine definierte Sollposition einstellbar ist. Dabei ist wesentlich, dass diese Sollposition immer gleich ist, dass also das Prüfgut entweder keine Auslenkung erfährt (Sollposition ist durch gespannte Normallage des Prüfguts definiert), oder zumindest immer eine gleich grosse Auslenkung.

Diese Verschiebbarkeit und Einstellbarkeit des Messkamms 8 wird dadurch erreicht, dass dieser auf einem Halteblock 16 montiert ist, an welchem ausserdem eine in der Verstellrichtung des Messkamms 8 orientierte Gewindespindel 17 befestigt ist. Der mit dem Gewinde versehene Teil dieser Gewindespindel ist in einem im Modul 7 (Fig. 1) ortsfesten Antriebsteil 18 gelagert, welcher einen Motor 19 und ein von diesem antreibbares Gewindeschloss (nicht dargestellt) aufweist. Bei Betätigungen des Motors 19 in der einen oder anderen Richtung wird das Gewindeschloss in Rotation in der entsprechenden Richtung versetzt und die Gewindespindel 17 mitsamt Halteblock 16 und Messkamm 8 wird in Richtung des Doppelpfeils A nach links oder nach rechts bewegt. Den verschiedenen Messspalten S1 bis S4 sind entlang der Gewindespindel 17 entsprechende Schalter T1 bis T4 zugeordnet, welche durch ein von der Gewindespindel 17 seitlich abstehendes Organ 20 betätigbar sind.

Im praktischen Betrieb des Gleichmässigkeitsprüfers wird von der Bedienungsperson in die Auswerteeinheit 2 (Fig. 1) der benötigte Messspalt S1, S2, S3, oder S4 eingegeben und dadurch der Motor 19 gestartet, der die Gewindespindel 17 so lange in Richtung des Pfeiles A verstellt, bis das Organ 20 den dem eingegebenen Messspalt zugeordneten Schalter T1, T2, T3 oder T4 kontaktiert. Da bei jeder Prüfung in die Auswerteeinheit 2 auch die Feinheit (Garnnummer) des Prüfguts eingegeben wird und zwischen dieser und dem geeigneten Messspalt eine eindeutige Beziehung besteht, kann die Auswahl des Messspalts auch automatisch anhand der eingegebenen Garnnummer erfolgen.

Vor jeder Prüfung wird das Prüfgut durch einen Einlegemechanismus (nicht dargestellt) in den entsprechenden Messspalt S1, S2, S3 oder S4 eingelegt. Anschliessend muss die Messeinheit 1 (Fig. 1) auf Null abgeglichen beziehungsweise muss der Nullabgleich überprüft werden. Zu diesem Zweck dient ein oberhalb des Messkamms 8 in Richtung der Tiefe der Messspalte angeordneter bügelartiger Garnheber 21, welcher auf einer in der genannten Richtung verstellbaren Gewindespindel 22 angeordnet ist. Diese Gewindespindel 22 ist ebenso wie die am Halteblock 16 befestigte Gewindespindel 17 in einem ortsfesten Antriebsteil 23 mit einem Motor 24 und einem Gewindeschloss (nicht dargestellt) gelagert. Bei Betätigung des Motors 24 wird die Gewindespindel 22 in Richtung des Doppelpfeils B nach vorne oder hinten bewegt, und dadurch wird das am Garnheber 21 anliegende Prüfgut entweder aus seinem Messspalt herausbewegt oder kann in diesen zurückgleiten. Die Gewindespindel 22 trägt ein seitlich abstehendes Organ 25, in dessen Bewegungsbahn zwei Schalter T5 und T6 angeordnet sind, bei deren Betätigung durch das Organ 25 der Motor 24 gestoppt wird. Der Start des Motors 24 und damit das Herausbewegen des Prüfguts aus dem Messspalt zum Zweck des Nullabgleichs erfolgt jeweils zwischen zwei Prüfungen oder am Ende einer Messreihe.

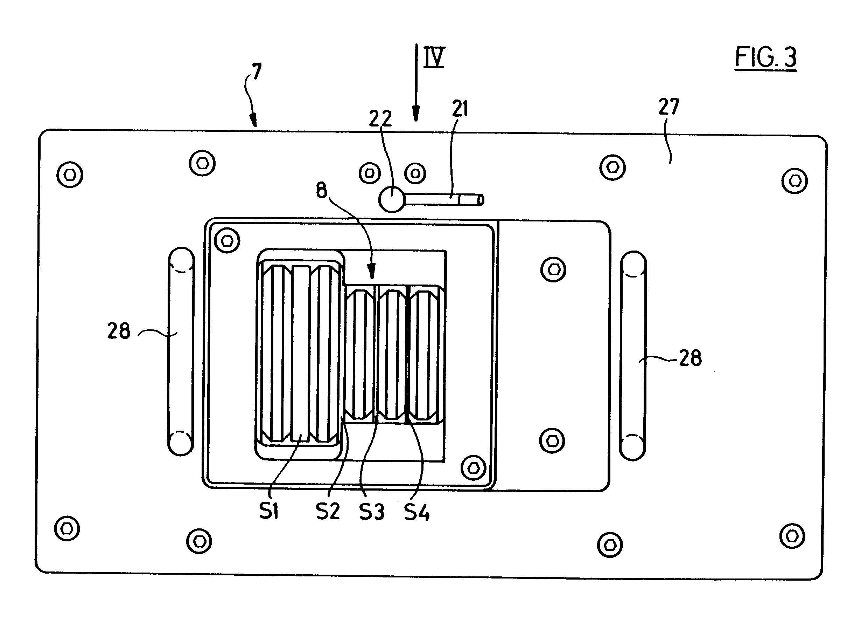

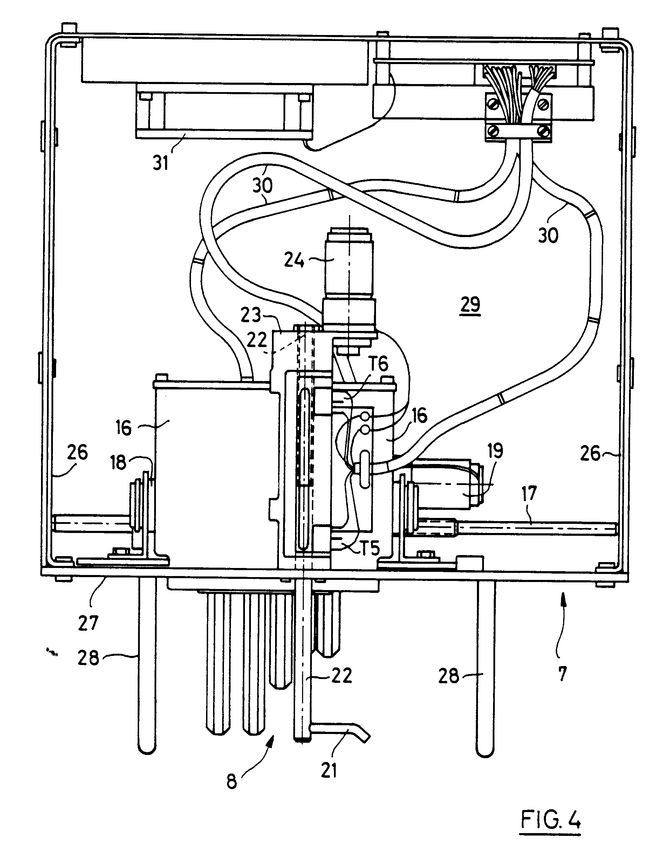

Aus den Fig. 3 und 4 ist die tatsächliche Anordnung der in Fig. 2 schematisch dargestellten Teile im Modul 7 (Fig. 1) ersichtlich. Das Modul 7 besteht darstellungsgemäss aus einem annähernd prismatischen Gehäuse 26, welches in den Rahmen 14 (Fig. 1) eingeschoben ist. Aus der Frontplatte 27 des Gehäuses 26 ragt der Messkamm 8 mit den darstellungsgemäss vier verschieden breiten Messspalten S1 bis S4 sowie der Garnheber 21. Zu beiden Seiten des Messkamms 8 ist je ein ebenfalls aus der Frontplatte 27 ragender bügelartiger Griff 28 für die Handhabung des Moduls 7 angeordnet.

An die Innenseite der Frontplatte 27 ist der Halteblock 23 angeschraubt, welcher einerseits die Gewindespindel 22 mit dem Garnheber 21 und anderseits den Motor 24 trägt. Der Halteblock 23 ist so positioniert, dass der Garnheber 21 in die Bewegungsbahn des zwischen der Fadenführungseinrichtung 6 und der Absaugdüse 12 gespannten Prüfguts P (Fig. 1) ragt.

Der Halteblock 18 für die Gewindespindel 17 liegt unterhalb des Halteblocks 23 und ist mit der Grundplatte 29 und mit der Frontplatte 27 des Moduls 7 verschraubt. Die Gewindespindel 17 ist im Halteblock 18 gelagert und erstreckt sich darstellungsgemäss über die gesamte Breite des Moduls 7. Das Gewindeschloss auf der Gewindespindel 17 ist mit dem oberhalb des Halteblocks 18 für die Gewindespindel 17 angeordneten Halteblock 16 mit dem Messkamm 8 verbunden, so dass bei Rotation des Gewindeschlosses der Halteblock 16 und damit der Messkamm 8 in Richtung der Gewindespindel 17 bewegt wird. Die verschiedenen elektrischen Leitungen 30 zum Messkamm 8, zu den Motoren 19 und 24 und zu den Schaltern T1 bis T6 sind zur Rückwand des Moduls 7 geführt. An dieser Rückwand ist ausserdem ein Ventilator 31 zur Kühlung und Belüftung des Innenteils des Moduls 7 montiert.

Wenn auch bei dem dargestellten Ausführungsbeispiel sowohl der Antrieb des Messkamms 8 als auch derjenige des Garnhebers 21 mittels eines Motors und einer Gewindespindel erfolgt, so können dafür selbstverständlich auch hydraulische oder pneumatische Antriebe verwendet werden. Besonders pneumatische Antriebe sind in der Textilindustrie stark verbreitet, so dass deren Anwendung für den Fachmann naheliegend ist.

## Patentansprüche

1. Vorrichtung zur automatischen Bestimmung von Kenngrössen von textilem Prüfgut (P), wie Garnen, Vorgarnen und Bändern, mit einer eine Führungseinrichtung (6), ein Messorgan (8), eine Vorschubeinrichtung (10) und ein Abzugsorgan (12) für das Prüfgut enthaltenden Messeinheit (1), deren Messorgan durch einen Messkamm mit Messspalten (S1 bis S4) verschiedener Breite gebildet ist, gekennzeichnet durch einen oberhalb des Messkamms (8) in Richtung der Tiefe der Messspalte (S1 bis S4) angeordneten bügelartigen Garnheber (21) und durch ein in der genannten Richtung (B) verstellbares Verstellorgan (22), auf dem der genannte Garnheber angeordnet ist.

2. Vorrichtung nach Anspruch 1, dadurch gekennzeichnet, dass der Messkamm (8) quer zur Laufrichtung des Prüfguts (P) verstellbar ausgebildet und jeder Messspalt (S1 bis S4) auf eine bestimmte Sollposition einstellbar ist, und dass der Garnheber (21) im Bereich dieser Sollposition angeordnet ist.

3. Vorrichtung nach Anspruch 2, dadurch gekennzeichnet, dass das den Garnheber (21) tragende Verstellorgan (22) durch eine Gewindespindel gebildet ist, die mit einem durch einen Motor (24) antreibbaren Gewindeschloss in Eingriff steht.

4. Vorrichtung nach Anspruch 3, dadurch gekennzeichnet, dass der Verstellweg der Gewindespindel (22) durch zwei Endpositionen begrenzt ist, in deren einer das Prüfgut (P) vollständig aus dem Messspalt (S1 bis S4) bewegt und in deren anderer das Prüfgut im Messspalt geführt und ausser Eingriff mit dem Garnheber (21) ist.

5. Vorrichtung nach Anspruch 4, dadurch gekennzeichnet, dass den beiden Endpositionen des Verstellwegs der Gewindespindel (22) zwei den das Gewindeschloss antreibenden Motor (24) steuernde Schalter (T5, T6) zugeordnet sind, und dass ein Organ (25) zur Betätigung dieser Schalter vorgesehen ist.

6. Vorrichtung nach Anspruch 3, dadurch gekennzeichnet, dass der bügelartige Garnheber (21) am einen Ende der Gewindespindel (22) angeordnet und quer zum Prüfgut (P) sowie parallel zur Verstellrichtung (A) des Messkamms (8) orientiert ist.

7. Vorrichtung nach Anspruch 6, dadurch gekennzeichnet, dass der Messkamm (8) und die Gewindespindel (22) in einem gemeinsamen Gehäuse (26) angeordnet sind, welches einen modulartigen Baustein der Messeinheit (1) bildet, und dass der Messkamm und das den bügelartigen Garnheber (21) tragende Ende der Gewindespindel aus der Frontplatte (27) dieses Gehäuses ragen.

8. Vorrichtung nach Anspruch 7, dadurch gekennzeichnet, dass das Prüfgut (P) den jeweiligen Messspalt (S1 bis S4) in vertikaler Richtung durchläuft, und dass die Gewindespindel (22) mit dem bügelartigen Garnheber (21) in Laufrichtung des Prüfguts vor dem Messkamm (8), vorzugsweise oberhalb von diesem, angeordnet ist.

## Claims

1. Apparatus for the automatic determination of characteristic magnitudes of textile material (P) to be tested, such as yarns, rovings and slivers, having a measuring unit (1) comprising a guide device (6), a measuring instrument (8), a feed device (10) and a draw-off device (12) for the test material, the measuring instrument being formed by a measuring comb with measuring gaps (S1 to S4) of different width, characterized by a stirrup-like yarn lifter (21) arranged above the measuring comb (8) in the direction of the depth of the measuring gaps (S1 to S4) and by a displacement device (22) adjustable in the said direction (B), the said yarn lifter being arranged on the displacement device.

2. Apparatus according to claim 1, characterized in that the measuring comb (8) is displaceable transversely to the direction of movement of the test material (P), each measuring gap (S1 to S4) being adjustable to a particular nominal position, and in that the yarn lifter (21) is arranged in the region of this nominal position.

3. Apparatus according to claim 2, characterized in that the displacement device (22) carrying the yarn lifter (21) is formed by a threaded spindle which engages with a threaded nut member driveable by a motor (24).

4. Apparatus according to claim 3, characterized in that the path of displacement of the threaded spindle (22) is limited by two end positions, in one of which the test material (P) is completely moved out of the measuring gap (S1 to S4) while in the other the test material is guided in the measuring gap and is out of engagement with the yarn lifter (21).

5. Apparatus according to claim 4, characterized in that two switches (T5, T6) controlling the motor (24) driving the threaded nut are associated to the two end positions of the path of displacement of the threaded spindle (22), and in that a member (25) for actuating the switches is provided.

6. Apparatus according to claim 3, characterized in that the stirrup-like yarn lifter (21) is arranged at the one end of the threaded spindle (22) and is aligned transversely to the test material (P) and parallel to the direction (A) of displacement of the measuring comb (8).

7. Apparatus according to claim 6, characterized in hat the measuring comb (8) and the threaded spindle (22) are arranged in a common housing (26) which forms a modular unit of the measuring unit (1), and in that the measuring comb and the end of the threaded spindle which carries the stirrup-like yarn lifter (21) project from the front panel (27) of this housing.

8. Apparatus according to claim 7, characterized in that the test material (P) passes vertically through the respective measuring gap (S1 to S4) and in that the threaded spindle (22) with the stirrup-like yarn lifter (21) is arranged upstream of the measuring comb (8) in the direction of movement of the test material, preferably above of the comb.

## Revendications

1. Dispositif pour la détermination automatique de caractéristiques d'un produit textile (P) tel que des fils, des mèches de préparation et des rubans, avec une unité de mesure (1) comprenant un dispositif de guidage (6), un organe de mesure (8), un dispositif d'avance (10) et un organe d'aspiration (12) du produit textile, dont l'organe de mesure est constitué par un peigne de mesure présentant des fentes de mesure (S1 à S4) de largeurs différentes, caractérisé par un élévateur de fil (21) en forme d'étrier disposé au-dessus du peigne de mesure (8) dans la direction de la profondeur des fentes de mesure (S1 à S4) et par un organe de réglage (22) déplaçable dans la direction indiquée (B) sur lequel est disposé l'élévateur de fil précité.

2. Dispositif selon la revendication 1, caractérisé en ce que le peigne de mesure (8) est réalisé de façon déplaçable transversalement au sens de la marche du produit textile (P), et en ce que chaque fente de mesure (S1 à S4) peut être réglée à une position de consigne définie, et en ce que l'élévateur de fil (21) est disposé au voisinage de cette position de consigne.

3. Dispositif selon la revendication 2, caractérisé en ce que l'organe de réglage (22) supportant l'élévateur de fil (21) est réalisé par une broche filetée qui est en prise avec un manchon à filetage interne pouvant être entraîné par un moteur (24).

4. Dispositif selon la revendication 3, caractérisé en ce que le trajet de déplacement de la broche filetée (22) est délimité par deux positions extrêmes dans l'une desquelles le produit textile (P) est sorti entièrement de la fente de mesure (S1 à S4) et dans l'autre desquelles le produit textile est guidé dans la fente de mesure et est hors prise avec l'élévateur de fil (21).

5. Dispositif selon la revendication 4, caractérisé en ce que deux commutateurs (T5, T6) commandant le moteur (24) entraînant le manchon à filetage interne sont associés aux deux positions extrêmes du trajet de déplacement de la broche filetée (22), et qu'un organe (25) est prévu pour l'actionnement de ces commutateurs.

6. Dispositif selon la revendication 3, caractérisé en ce que l'élévateur de fil (21) en forme d'étrier est disposé à une extrémité de la broche filetée (22) et est orienté transversalement au produit textile (P) ainsi que parallèlement à la direction de déplacement (A) du peigne de mesure (8).

7. Dispositif selon la revendication 6, caractérisé en ce que le peigne de mesure (8) et la broche filetée (22) sont disposés dans un boîtier commun (26) qui constitue un bloc fonctionnel en forme de module de l'unité de mesure (1), et en ce que le peigne de mesure et l'extrémité de la broche filetée supportant l'élévateur de fil (21) en forme d'étrier font saillie de la plaque avant (27) de ce boîtier.

8. Dispositif selon la revendication 7, caractérisé en ce que le produit textile (P) traverse la fente de mesure respective (S1 à S4) dans la direction verticale, et en ce que la broche filetée (22) avec l'élévateur de fil en forme d'étrier (21) est disposé dans le sens de la marche du produit textile devant le peigne de mesure (8), de préférence au-dessus de celui-ci.
